# EUROPEAN PATENT APPLICATION

(11) **EP 2 589 404 A1**
(43) Date of publication of application: **08.05.2013**
(21) Application number: 11188027.4
(22) Date of filing: 07.11.2011
(51) Int. Cl.: A61M 16/06, A61B 5/083

(54) **Breathing mask for ventilating a patient and gas analyzer for respiratory gas measurement**

(71) Applicant: General Electric Company, Schenectady, NY 12345 (US)
(72) Inventor: Haveri, Heikki Antti Mikael, 03150 Huhmar (FI)
(74) Representative: Kanerva, Arto

(57) **Abstract**

A breathing mask (3) for ventilating a patient is disclosed herein. The breathing mask includes a housing (15) covering a respiratory passage of the patient and a first cavity (25) surrounded at least partly by the housing conveying both an inspiratory and expiratory gas. The breathing mask further includes a respiratory port (32) delivering the inspiratory gas from the first cavity (25) and the expiratory gas to the first cavity (25) and a sampling cell (26) in flow communication with the first cavity, which sampling cell is adapted to convey both the inspiratory gas towards the respiratory port and the expiratory gas from the respiratory port. The sampling cell comprises at least one component (33) for enabling a signal acquisition of at least one of the inspiratory and expiratory gas compound. A gas analyzer for respiratory gas measurement is also provided.

## Description

### BACKGROUND OF THE INVENTION

This disclosure relates generally to a breathing mask for ventilating a patient and a gas analyzer for respiratory gas measurement.

Tidal volume (TV) is an amount of an air inspired or taken into the lungs in a single breath. TV is also dependent on the sex, size, height, age and a health etc. of a patient. In general TV also decreases as the size of the patient decreases. In an average healthy adult, TV is about 400-600 ml whereas in an average healthy neonate, that measures 3.5-4 kg and is 50 cm tall, TV is approximately 25-50 ml. On the other hand, in an average premature neonate that measures only 500 grams TV is only about 2-3.5 ml. TV of a smaller patient's is very difficult to measure, but it can be approximated to 4-7 ml/kg, applying a general rule of thumb for approximating the TV of the human lung. In practice the TV of the patient suffering pulmonary system deficiency is normally less than the approximation gives.

When the patient is mechanically ventilated with a conventional ventilator, an endotracheal tube is placed into a trachea so that it goes through oral or nasal cavity and larynx. In tracheostomy endotracheal tube goes straight into trachea through neck. The other end of the endotracheal tube is connected to a breathing circuit Y-piece through a luer type connector. If the patient is gas monitored with a mainstream or sidestream gas analyzer, an airway adapter used for sampling the breathing gas that is analyzed by the gas analyzer is normally connected between connectors of the endotracheal tube and the breathing circuit Y-piece.

Continuous Positive Airway Pressure (CPAP) is a non-invasive positive pressure ventilation used to maintain an elevated baseline respiratory system pressure during spontaneous breathing. Neonates or infants are preferential nose breathers until 5 months of age, which easily facilitates the application of nasal CPAP for a variety of clinical conditions including respiratory distress syndrome, apnea of prematurity and in other conditions that require positive pressure. This is accomplished by inserting nasopharyngeal tubes, affixing nasal prongs, or fitting a nasal mask to the patient.

Continuous flow CPAP systems use a preset flow of gas to maintain CPAP through nasal prongs. CPAP delivered is dependent upon the flow rate and the resistance created by the exhalation valve that is housed in the breathing circuit. Typically, ventilator-driven CPAP represents a continuous flow system. Because of the presence of an exhalation valve, patients must exhale against a fixed resistance, thus resulting in a higher induced work of breathing.

Variable flow CPAP technology incorporates a flow driver that delivers fresh gas through a breathing circuit to a dual injector generator with a specially designed valve, mask or nasal prongs. Gas enters at the point of the interface on inspiration and shunts flow away through an expiratory gas channel as the patient desires on exhalation. CPAP levels are stabilized and maintained by a change in the flow rate at the generator with little variability, unless there is a leak at the patient's interface.

Bi-level CPAP, known by different acronyms such as SiPAP™ or biphasic CPAP or nasal BIPAP, is another type of CPAP that allows spontaneous breathing at two levels of CPAP. A sigh level of CPAP is reached for a preset defined interval of time (set as inspiratory time) and a low level, the baseline CPAP is maintained continuously. The number of sighs is determined and preset by the caregiver. The difference created by these two levels of pressure is minimal, however; it may be associated with small changes in volume and associated increases in functional residual capacity (FRC), which can be integral to recruitment. The goal of this bi-level CPAP is to achieve some higher level of alveolar recruitment and prevent alveolar collapse.

Synchronized non-invasive positive pressure ventilation (SNIPPV) is another method to deliver positive pressure breaths through nasal prongs or a mask. In SNIPPV, gas flow issues from a flow driver used in variable flow CPAP or through a mechanical ventilator. SNIPPV has been shown to decrease work of breathing in infants with respiratory distress syndrome compared to nasal CPAP alone.

Synchronization to the breathing cycle can be done various ways. It is common to measure the breathing circuit pressure and/or flow during the breathing cycle from the breathing circuit at the ventilator. In this case the distance between the patient and the ventilator is longer, which may induce disturbances to the measurement and decreases the sensitivity, as well as to the synchronization. Some ventilators support a flow measurement where a flow sensor is attached closer to the patient usually between the breathing circuit and the accessory attached to the patient. This type of measurement usually functions more accurately, but adds dead space or volume that causes rebreathing of gases. It is also possible to measure a diaphragm or chest movement with additional sensors to support ventilator synchronization. The movement can be measured with piezo-electric or resistive, strain gauge or similar based sensors externally from the chest by attaching the sensor on the skin or even internally from for example through a stomach with a catheter that comprises a measurement sensor. An electrical signal from the sensor(s) is then transmitted into the ventilator where it is used to synchronize for example the pressure support, thus to detect patient's sigh to deliver higher pressure within inspiration and lower pressure during expiration.

Common for all ventilation methods is that during an inspiration the fresh breathing gas comprising higher oxygen (O₂) concentration should flow into the patient's lungs through a breathing circuit, nasopharyngeal tubes, affixing nasal prongs, or a nasal mask, then to oral or nasal cavities, a trachea, a bronchus, a bronchi, bronchioles and finally reaching an alveoli deep in the lungs, where all the gas exchange actually occurs. Carbon dioxide (CO₂) molecules in hemoglobin of a blood flowing in tiny blood vessels around the alveoli are replaced with O₂ molecules in the fresh breathing gas through the thin walls of the alveoli. O₂ molecules take their place in the hemoglobin, whereas CO₂ molecules flow out from the patient within the used expired breathing gas, through the same path as the fresh gas came in during the inspiration. This path common for inspiratory and expiratory gases inside the patient's respiratory system causes rebreathing of gases and is called anatomical dead volume.

The anatomical dead volume is almost impossible to reduce, but it is proportional to the size and the physical condition of the patient. The mechanical dead volume depends on a breathing circuit design, an inner diameter of a tubing, connectors and additional accessories, such as sidestream and mainstream gas analyzers connected to patient's respiratory system. Obviously the mechanical dead volume is more critical for smaller patients with smaller TV or patients suffering barotraumas etc., which also decrease TV. In practice the mainstream and the sidestream gas analyzing is not suitable for the patients with very small TV due their large dead space. In addition to a dead volume increment caused by the airway adapter, conventional sidestream gas analyzers "steal" sample gas from the inspiratory and expiratory gas flow, thus decreasing the gas exchange in the alveoli even more. Furthermore respiration rates (RR) of smaller patients are higher, up to 150 breaths/minute or even more, which is well above the measurement range of the conventional gas measurement technology, compared to adult patients with RR usually much less than 60 breaths/minute.

At the moment there does not exist a suitable and reliable, noninvasive, real time gas concentration measurement for smaller patients, especially for neonates to ensure that the gas exchange in the lungs, between the blood and air interface really occurs. It is sometimes recommended to use transcutaneous gas concentration measurement with CPAP, at least to reduce the risk of inadequate ventilation, for example caused by a ventilator malfunction, such as a leaking mask etc. However, transcutaneous measurement is expensive, unreliable, needs complicated calibration routines and it is slow as it measures the blood gas concentration through the patient's skin. The sensor is attached to skin with a tape or adhesive and is heated up to over 40°C that easily causes a skin damage to sick patients due to burning, especially for example to very delicate and fragile skin of smaller patients. A problem with existing non-invasive gas analyzers, especially mainstream type analyzers, is the very large dead space or dead volume that causes rebreathing of gases. Existing mainstream type gas analyzers comprise a separate airway adapter, which is designed for use with intubated patients. It is common to make attempts to connect this airway adapter in series with the breathing mask and the branching unit, used to deliver inspiratory and expiratory gases, to enable the gas concentration measurement. However, the dead space in addition with non-tubular breathing gas flow path and large dead volume of the mask deteriorate the gas concentration measurement as the inspiratory and expiratory gases are mixed and averaged. Thus the measured gas concentration values do not correspond to values of blood gases. Thus the only reliable method to find out a correct gas concentration in the blood is to take blood samples and analyze them in a laboratory at the moment. This is slow, expensive, unpleasant and hazardous for the patient of course. Blood sampling causes infection risk, neural damages and if used in longer term it may end up to blood transfusions.

### BRIEF DESCRIPTION OF THE INVENTION

The above-mentioned shortcomings, disadvantages and problems are addressed herein which will be understood by reading and understanding the following specification.

In an embodiment a breathing mask for ventilating a patient includes a housing adapted to cover a respiratory passage of the patient and a first cavity surrounded at least partly by the housing adapted to convey both an inspiratory and expiratory gas. The breathing mask for ventilating a patient also includes a respiratory port adapted to deliver the inspiratory gas from the first cavity and the expiratory gas to the first cavity, and a sampling cell in flow communication with the first cavity, which sampling cell is adapted to convey both the inspiratory gas towards the respiratory port and the expiratory gas from the respiratory port. The sampling cell comprises at least one component for enabling a signal acquisition of at least one of the inspiratory and expiratory gas compound.

In another embodiment a gas analyzer for respiratory gas measurement includes a breathing mask for ventilating a patient including a housing adapted to cover a respiratory passage of the patient, a first cavity surrounded at least partly by the housing for conveying both an inspiratory and expiratory gas, and a respiratory port adapted to deliver the inspiratory gas from the first cavity and the expiratory gas to the first cavity. The gas analyzer for respiratory gas measurement also includes a sensor connectable to the breathing mask, the sensor including a radiation source adapted to emit a radiation, at least one detector adapted to receive the radiation emitted by the radiation source and providing a signal indicative of at least one of the inspiratory and expiratory gas compound, and an electronics board adapted to process the signal received from the at least one detector, The breathing mask further including a sampling cell in flow communication with the first cavity, which sampling cell is adapted to convey both the inspiratory gas towards the respiratory port and the expiratory gas from the respiratory port. The sampling cell includes at least one component for enabling a signal acquisition of at least one of the inspiratory and expiratory gas compound.

In yet another embodiment, a breathing mask for ventilating a patient includes a housing adapted to cover a respiratory passage of the patient, and a first cavity surrounded at least partly by the housing for conveying both an inspiratory and expiratory gas. The breathing mask for ventilating a patient also includes a first port adapted to deliver inspiratory gas to the first cavity, a second port adapted to deliver expiratory gas from the first cavity, and a respiratory port adapted to deliver the inspiratory gas from the first cavity and the expiratory gas to the first cavity. The breathing mask for ventilating a patient further includes a sampling cell in flow communication with the first cavity, which sampling cell is adapted to convey both the inspiratory gas from the first port to the respiratory port and the expiratory gas from the respiratory port to the second port. The sampling cell includes at least one component for enabling a signal acquisition of at least one of the inspiratory and expiratory gas compound.

Various other features, objects, and advantages of the invention will be made apparent to those skilled in art from the accompanying drawings and detailed description thereof.

### BRIEF DESCRIPTION OF THE DRAWINGS

Figure 1 shows a schematic view of a breathing circuit including a breathing mask and a gas analyzer in accordance with an embodiment;

Figure 2 shows a cross-sectional schematic view of the breathing mask in Figure 1 seen from the side and above;

Figure 3 shows a cross-sectional schematic side view of the breathing mask in accordance with another embodiment;

Figure 4 shows a cross-sectional schematic side view of the sensor in Figure 1; and

Figure 5 shows a cross-sectional schematic side view of the breathing mask in accordance with another embodiment

### DETAILED DESCRIPTION OF THE INVENTION

Specific embodiments are explained in the following detailed description making a reference to accompanying drawings. These detailed embodiments can naturally be modified and should not limit the scope of the invention as set for in the claims.

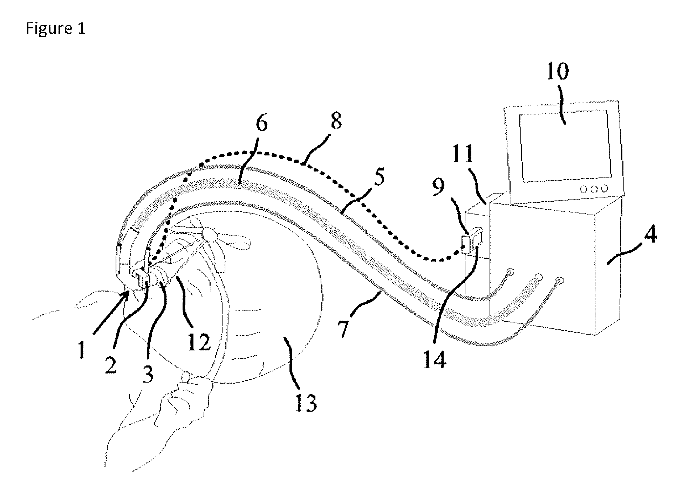

Figure 1 show a new mainstream type breathing gas analyzer 1, that comprises a sensor 2 connectable to a breathing mask 3, such as a nasal mask, for ventilating a patient, which sensor further connects to a host device 4, which may be a ventilator in this particular embodiment, through an inspiratory tube 5 that allows inspiratory gas, such as fresh gas, to flow into the patient's lungs, an expiratory tube 6 that allows expiratory gas to flow out from the patient's lungs and connection 7 that allows the ventilator to be synchronized with patient's respiratory cycle. The breathing mask 3 for neonates and possibly for pediatrics covering only nostrils without the mouth is attachable around or against nostrils, but there are breathing masks for different patient groups attachable around at least one respiratory passage of the patient, when nostrils of a nose is one passage and a mouth is another passage. The breathing mask for neonates is attached against patient's nostrils and kept in place with a flexible string 12 attached to a cap 13 or similar.

The sensor 2, which is detachable from the breathing mask 3, can also be connected electrically to the host device 4 through a cable 8 and connector 9. The host device can transfer electric power through the connector 9 and the cable 8 into the sensor 2 to operate the sensor. The sensor can also be wireless in which case it comprises a battery (not shown in figures) to operate the sensor. The sensor 2 can transmit the measurement or signal data and receive control data through radio frequency transceiver or similar between connector 9 or between a module 11 that also comprise a radio frequency transceiver or similar to receive and transmit the data. The connector 9 or module 11 may comprise a processing unit 14, such as a cental processing unit (CPU), or it can be located into the sensor 2 to operate the sensor and to make one of qualitative and quantitative gas compound analysis of at least one of the inspiratory and expiratory gas based on the signal received from the detector. Also the processing unit 14 may determine or calculate besides gas compounds and gas concentration values, but also pressures, flows etc. Vice versa the at least one of the inspiratory and expiratory gas property calculated by the processing unit can be transferred from the sensor 2 to the host device 4 to show the information for the care giver or user on a display 10 or similar.

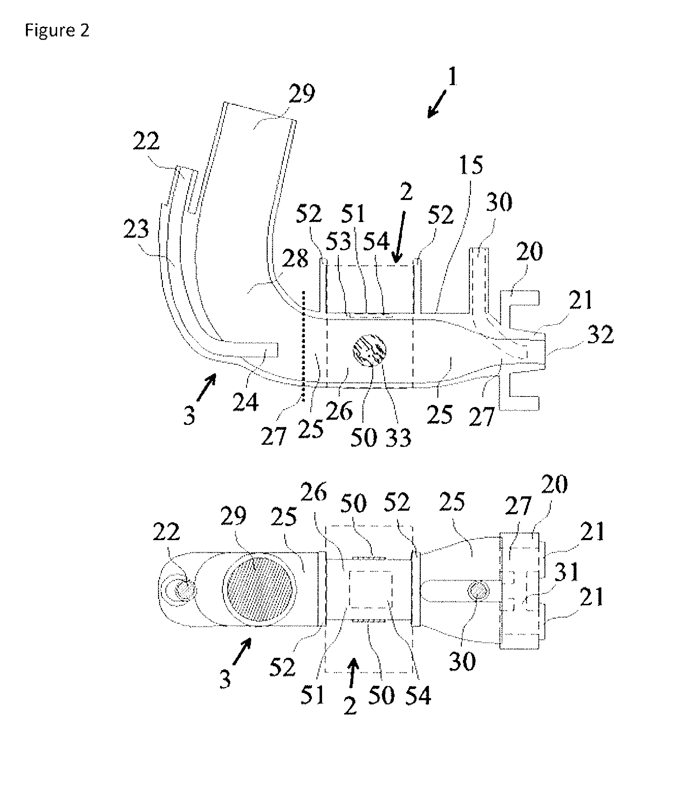

Figure 2 shows two, more detailed, schematic cross-sectional views of one embodiment of the breathing mask 3 for neonates. An upper view is from the side and a lower view is from above. This breathing mask 3 comprises a housing 15 and a first cavity 25, which housing covers at least one of the respiratory passages, which is in this specific case the nostrils, and which housing surrounds a first cavity 25. In this embodiment there is also a first port 22 to deliver an inspiratory gas to the first cavity 25, a second port 29 to deliver expiratory gas from the first cavity 25 and a respiratory port 32 to deliver the inspiratory gas from the first cavity 25 to the respiratory passage and the expiratory gas from the respiratory passage to the first cavity 25. The breathing mask 3 also comprises a gas sampling cell 26 in flow communication with the first cavity to convey both the inspiratory gas from the first port 22 to the respiratory port 32 and the expiratory gas from the respiratory port to the second port 29. The gas sampling cell 26 integrated into the breathing mask 3 and the first cavity 25 are advantageously in direct flow communication with each other. The sampling cell 26 comprises at least one component 33 for enabling a signal acquisition of at least one of the inspiratory and expiratory gas compound, which signal may be needed while making one of qualitative and quantitative gas analysis of at least one respiratory gas compound.

The mask for neonates may further comprise a respiratory passage piece 20, such as a nose piece, made of elastic material such as silicone. The piece 20 in Figure 2 is placed against the patient's nostrils so that tubes 21 enter the both nostrils to distribute inspiratory gas from the first cavity 25 and expiratory gas to the first cavity 25 to prevent the inspiratory or expiratory gas from leaking. The inspiratory gas is delivered through the first port 22 into an input cavity 23 and delivered or injected from inspiratory output 24 towards the patient's nostrils, first into the first cavity 25 common for inspiratory and expiratory gas and through the gas sampling cell 26 between the first cavity 25 and then to nostrils through the tubes 21 of the respiratory port 32. Expiratory gas from patient's lungs flows the same path, but into the opposite direction, first through the tubes 21 of the respiratory port 32, then first cavity 25 and sampling cell 26 between the first cavity 25, but then by inspiratory output 24, through a second cavity 28, such as an output cavity, and out from the breathing mask 3 through the second port 29.

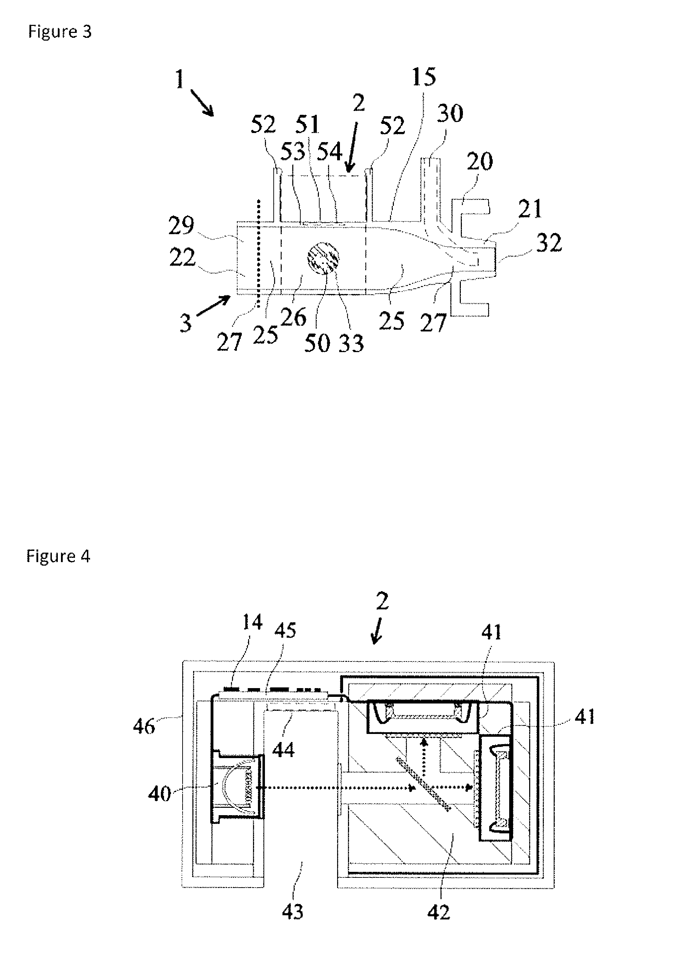

It should be noted that in the breathing mask 3 the same port for example at the position, where the dashed line 27 shown in Figure 2 divides the first cavity 25, may function as the first port 22 for the inspiration gas and as the second port 29 for the expiration gas as shown in Figure 3 depending on a phase of the breathing cycle. This means that the first port 22 and the second port 29 can physically be the same port but functionally different ports. In that case the design would slightly differ from the design in Figure 2 when a dashed line 27 divides the mask 3 so that a branching unit with the second cavity 28 and the input cavity 23 on the left hand side of this line has been detached into a separate part, which does not necessarily be included in the breathing mask.

The sampling cell 26 may comprise a pressure cavity 30, which connects with the tubes 21 of the respiratory port 32 through a gateway 31 and which is used for acquiring a signal indicative of a pressure to determine the pressure during breathing cycle in the embodiment in Figure 2, which information is used by the host device 4 to synchronize the start of delivery of inspiratory air with the start of patient's inspiration.

Other methods to synchronize the start of delivery of inspiratory air with the start of patient's inspiration can be used as well. If for example the breathing pressure is measured by the host device 4 through the expiratory tube 6 or if a diaphragm or chest movement is measured internally or externally with electrical sensors sensitive to mechanical movement the pressure cavity 30 and gateway 31 can be left out to simplify the breathing mask 3 in Figure 2.

The sensor 2 shown in Figure 4 comprises a radiation source 40, such as an infrared radiation source, at least one detector 41, such as a thermal detector, and an electronics board 45, which may also include the processing unit 14. The radiation source, the detector and the electronics board may be attached to the body 46, typically they are inside the body 46. The radiation emitted by the radiation source 40 is directed towards the at least one detector 41 placed inside a thermal mass 42 of the sensor 2 on the other side of a sensor cavity 43. Thus the detector and the radiation source may locate on opposite sides of the sensor cavity 43. As explained hereinbefore the gas sampling cell in Figure 2 comprises at least one component 33 for enabling a signal acquisition of at least one of the inspiratory and expiratory gas compound. The component 33 in the embodiment with the radiation source and the detector is at least one optical window 50, but typically there are two optical windows placed to the sides of the sampling cell 26.

The sensor 2 connects with the breathing mask 3 so that the cavity 43 of the sensor 2 slides on the sampling cell 26 of the breathing mask 3 so that the first surface 44 of the cavity 43 in the sensor 2 is towards the second surface 51 of the sampling cell 26. When the first surface 44 of the cavity 43 reaches the second surface 51 of the sampling cell 26 the sensor 2 locks between wings 52 in the breathing mask 3 and the optical windows 50 on both sides of the sampling cell 26 are aligned with the radiation source 40 and the at least one detector 41 in body 46 of the sensor. Now the radiation from the radiation source 40 can traverse through the optical windows 50 and through the breathing gas flowing crosswise through the sampling cell 26. Different gases flowing through the sampling cell 26 absorb infrared radiation at certain wavelengths that can be measured with the at least one detector 41. The detector transforms the received radiation into a signal, such as voltage(s), which is proportional to the concentration of at least one measured gas. The signal is further processed in the electronics board 45 and transmitted to the host device 4, the connector 9 or the module 11.

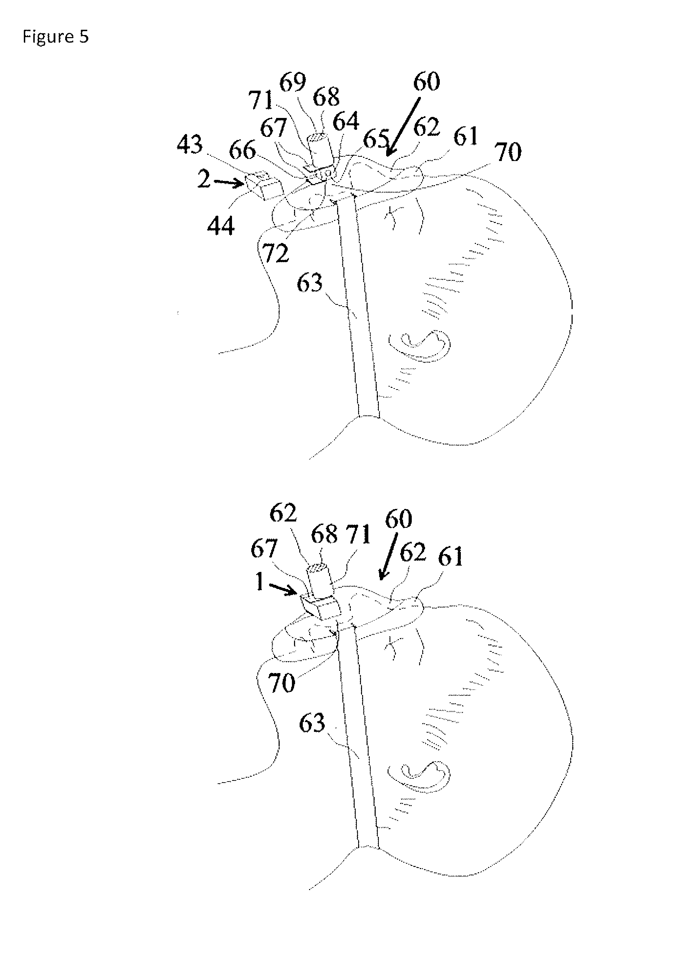

Figure 5 shows another embodiment of a breathing mask 60, such as a face mask covering both the nostrils and the mouth, with the sensor 2. An upper view is from the side when the sensor is detached from the breathing mask and a lower view, which is also from the side, the sensor 2 is connected to the breathing mask 60. The breathing mask masks 60 can have different size modifications for different size of patients, but breathing masks attachable to the face are more commonly used with larger patients since larger patients, especially adults and sometimes pediatrics, are not preferential nose breathers like neonates or infants. The breathing mask 60 for the face connects and seals around patient's nose and mouth through very elastic tubular shaped portion, a cushion 61, which may be air filled in this particular case, which circulates around a stiffer housing 62 of the breathing mask 60. An elastic string 63 circulating around the patient's neck and/or the head tighten and keep the breathing mask on its place.

The housing 62 comprises the gas sampling cell 64 integrated to the housing 62 as shown in Figure 5. The gas sampling cell comprises at least one component 72 for enabling acquisition a signal indicative of at least one of the inspiratory and expiratory gas compound. The component is in this embodiment the optical window 65 or actually there are two parallel optical windows 65 placed to the sides of the sampling cell 64. The sensor 2 as described earlier and was shown in Figure 4 connects with the breathing mask 60 so that the cavity 43 in the body of sensor 2 slides on the sampling cell 64 of the breathing mask 60 so that the first surface 44 of the cavity 43 in the sensor 2 is towards the second surface 66 of the sampling cell 60. When the first surface 44 of the cavity 43 reaches the second surface 66 of the sampling cell 60 the sensor 2 locks between the wings 67 in the breathing mask 60 and the optical windows 65 on the sides of the sampling cell 64 are aligned with the radiation source 40 and the detector(s) 41 in the body 46 of the sensor 2 as shown in Figure 4. Now the radiation from the radiation source 40 can traverse through the optical windows 65 and through the breathing gas flowing crosswise through the sampling cell 64. Different gases flowing through the sampling cell 64 absorb infrared radiation at certain wavelengths that can be measured with the at least one detector 41. The detector transforms the received radiation into a signal, such as voltage(s), which is proportional to the concentration of at least one measured gas compound. The signal is further processed in the electronics board 45 and transmitted to the host device 4, the connector 9 or the module 11. The sampling cell 64 is located close to and between the nostrils and the mouth in housing 62.

The housing 62 is shaped to direct expiratory breathing gases from the nostrils and/or the mouth through the respiratory port 70 through the sampling cell 64 to the first cavity 71 and out from the second port 68. The direction of respiratory port 70, sampling cell 64 and the first cavity 71 is away from patient's nose and/or mouth so that breathing gas from the nostrils or mouth flows straight through the sampling cell 64 and do not circulate inside the mask 60. The second port 68 for the expiration gas connects the breathing mask 60 into the breathing circuit and ventilator (not shown in Figure 5). The ventilator also delivers inspiratory gas through the first port 69 to the breathing mask 60 and through the first cavity 71, the sampling cell 64 and the respiratory port 70 into the patient's lungs. It should be noted that in the breathing mask 60 covering both respiratory passages, which are the mouth and the nostrils of the nose, the same port may function as the first port 69 for the inspiration gas and as the second port 68 for the expiration gas. This means again that the first port 69 and the second port 68 can physically be the same port but functionally different ports. This port may be connected to a branching unit, such a Y-piece, (not shown in Figure 5) for separating inspiratory and expiratory gas flows between the breathing mask 60 and a host device 4, which may be in this embodiment the ventilator.

The breathing masks 3 and 60 can be reusable or disposable and they can be made of plastic, silicon, a combination of those or any other similar materials. The used materials and the construction of disposable accessory need to be recyclable and low cost to enable reasonable and efficient disposability. Some parts of the breathing mask can be reusable and include disposable parts that are changed more often, like the respiratory passage piece 20 of the breathing mask 3 for neonates shown in figure 2, which is made of very elastic material to fit patient's nostrils without causing harm, but which can be changed more often to reduce contamination or blockage risk due secretions. The passage piece 20 can also have different sizes to correspond better the size of a patient it is connected to. The breathing mask 3 can have less sizes, for example one for a group of neonates and one for a group of pediatrics, but within each group the final fitting to correspond the particular face and size of each patient is done by choosing the correct nose piece 20. The breathing masks 60 for larger patients can also have different sizes to fit better different size of patients.

The breathing masks 3 or 60 comprising the sampling cell 26 or 64 have several advantages compared to well-known masks. First of all it enables accurate, real time, non-invasive breathing gas concentration measurement of neonatal patients that do not exist at the moment. When the breathing mask and the sampling cell are integrated the dead volume can be remarkably minimized, which is especially important for neonatal and pediatrics, but also adults may have benefit for the integration. The dead volume may be decreased more than 10 ml with adults and more than 5 ml with pediatrics. The overall dead volume for neonates is less than 3 ml, but may be even less than 1 ml depending on the features integrated into the mask, such as pressure cavity to detect breathing cycle. In addition to decreased dead volume the integration of the mask and the gas analyzer enables very tubular flow path design for breathing gases that enables laminar flow of gases from the patient through the analyzer, in other words turbulences mixing the inspiratory and expiratory air are minimized to enable accurate analyzing of inspiratory air and expiratory air separately. This is very important in getting gas concentration measurement values that correlate with the blood gas concentration values, even when the respiration rate (RR) is increased. Any breathing gas analyzes would otherwise be useless.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A breathing mask (3; 60) for ventilating a patient comprising:
a housing (15; 62) adapted to cover a respiratory passage of the patient;
a first cavity (25; 71) surrounded at least partly by said housing adapted to convey both an inspiratory and expiratory gas; and
a respiratory port (32; 70) adapted to deliver the inspiratory gas from said first cavity (25; 71) and the expiratory gas to said first cavity (25);
**characterized in that**, said breathing mask further comprising a sampling cell (26; 64) in flow communication with said first cavity, which sampling cell is adapted to convey both the inspiratory gas towards said respiratory port and the expiratory gas from said respiratory port and which sampling cell comprises at least one component (33; 72) for enabling a signal acquisition of at least one of the inspiratory and expiratory gas compound.

2. The breathing mask according to claim 1, **characterized in that**, said at least one component (33; 72) is at least one optical window (50; 65).

3. The breathing mask according to claim 1 further comprising a pressure cavity (30) adapted to connect with said respiratory port (32) for acquiring a signal indicative of a pressure during a breathing cycle.

4. The breathing mask according to claim 1, further comprising a respiratory passage piece (20) adapted to be placed against nostrils and tubes (21) in said respiratory port (32) adapted to enter both nostrils to distribute inspiratory gas from said first cavity (25) and expiratory gas to said first cavity (25).

5. The breathing mask according claim 1, further comprising a first port (22; 69) adapted to deliver the inspiratory gas to said first cavity (25) and a second port (29; 68) adapted to deliver the expiratory gas from said first cavity (25;71).

6. The breathing mask according claim 5 further comprising an input cavity (23) and an inspiratory output (24), said input cavity locating between said first port (22) and said inspiratory output (24) delivering the inspiratory gas to said first cavity common for inspiratory and expiratory gas and through said sampling cell (26) and tubes (21) of said respiratory port (32) to nostrils.

7. The breathing mask according to claim 6 further comprising a second cavity (28) between said second port (29) and said inspiratory output (24) adapted to deliver expiratory gas from said first cavity (25;71) to said second port bypassing said inspiratory output.

8. The breathing mask according to claim 2, **characterized in that**, both sides of said sampling cell (26; 64) are provided with optical windows (50; 65) for enabling signal acquisition of at least one of the inspiratory and expiratory gas compound.

9. The breathing mask according to claim 8, **characterized in that**, the radiation is adapted to traverse through the optical windows and through the breathing gas flowing crosswise through said sampling cell.

10. The breathing mask according to claim 5, **characterized in that**, said first port (22) and said second port (29) can physically be the same port but functionally different ports.

11. A gas analyzer for respiratory gas measurement comprising;
a breathing mask (3; 60) for ventilating a patient comprising a housing (15; 62) adapted to cover a respiratory passage of the patient, a first cavity (25; 71) surrounded at least partly by said housing for conveying both an inspiratory and expiratory gas, and a respiratory port (32; 70) adapted to deliver the inspiratory gas from said first cavity (25;71) and the expiratory gas to said first cavity (25); and
a sensor (2) connectable to said breathing mask (3; 60), said sensor comprising a radiation source (40) adapted to emit a radiation, at least one detector (41) adapted to receive the radiation emitted by said radiation source and providing a signal indicative of at least one of the inspiratory and expiratory gas compound, and an electronics board (45) adapted to process the signal received from said at least one detector,
**characterized in that**, said breathing mask further comprising a sampling cell (26; 64) in flow communication with said first cavity, which sampling cell is adapted to convey both the inspiratory gas towards said respiratory port and the expiratory gas from said respiratory port and which sampling cell comprises at least one component (33;72) for enabling a signal acquisition of at least one of the inspiratory and expiratory gas compound.

12. The gas analyzer according to claim 11, **characterized in that**, said at least one component (33; 72) is at least one optical window (50; 65).

13. The gas analyzer according to claim 11, **characterized in that**, said breathing mask further comprising a pressure cavity (30) adapted to connect with said respiratory port (32) for acquiring a signal indicative of a pressure during a breathing cycle.

14. The gas analyzer according to claim 12, **characterized in that**, when said sensor and said sampling cell are connected, said optical windows (50; 65) are adapted to be on sides of said sampling cell (26; 64) and are adapted to be aligned with said radiation source (40) and said detector (41).

15. The gas analyzer according to claim 11, **characterized in that** said sensor is detachable from said breathing mask.
